# EUROPEAN PATENT SPECIFICATION

(11) **EP 4 065 154 B1**
(45) Date of publication and mention of the grant of the patent: **20.05.2026**
(21) Application number: 20820795.1
(22) Date of filing: 23.11.2020
(51) Int. Cl.: A61K 38/17, A61P 35/00

(54) **UK 114 SALMON PROTEINS FOR USE IN THE TREATMENT, DIAGNOSIS AND PREVENTION OF MALIGNANT TUMOURS**
UK-114-LACHSPROTEINE ZUR VERWENDUNG BEI DER BEHANDLUNG, DIAGNOSE UND VORBEUGUNG VON MALIGNEN TUMOREN
PROTÉINES DE SAUMON UK 114 DESTINÉES À ÊTRE UTILISÉES DANS LE TRAITEMENT, LE DIAGNOSTIC ET LA PRÉVENTION DE TUMEURS MALIGNES

(30) Priority: 26.11.2019 IT 201900022203
(43) Date of publication of application: 05.10.2022
(73) Proprietor: Bartorelli Cusani, Alberto, 98000 Monte Carlo (MC)
(72) Inventor: Bartorelli Cusani, Alberto, 98000 Monte Carlo (MC)
(74) Representative: Bianchetti & Minoja with Trevisan & Cuonzo IPS SRL
(86) International application number: PCT/EP2020/083069
(87) International publication number: WO 2021/105059

(56) References cited:
- WO-A1-2018/109096
- WO-A1-96/14340
- RACCA S ET AL: "Growth inhibition of DMBA-induced rat mammary carcinomas by UK 114", VIRCHOWS ARCHIV, SPRINGER INTERNATIONAL, BERLIN, DE, vol. 431, no. 5, 1 November 1997 (1997-11-01), pages 323 - 328, XP002267961, ISSN: 0945-6317, DOI: 10.1007/S004280050106

## Description

The present invention relates to UK 114 salmon proteins for use in the treatment and prevention of malignant tumours.

### PRIOR ART

The family of proteins called YjgF/YERO57c7/UK114, which are highly conserved in various prokaryotic and eukaryotic organisms, has formed the subject of numerous studies designed to investigate their structural and functional characteristics (Bartorelli et al. J. Tumor and Marker Oncology, 1994, 9, 37; Lambrecht JA et al., J.Biol. Chem., 285, 34401-34407; Lambrecht JA et al., J.Biol. Chem. 2012, 287, 3454-3461; Mistiniene E et al, Bioconjugate Chem. 2003, 14, 1243-1252; Dhawan L et al., Mol. Cell. Biol., 2012, 32, 3768-3775; Flynn JM et al., Mol. Microbiol., 2013, 89(4)751-759; Ernst DC et al., J. Bacteriology, 2014, 196 (18), 3335-3342; Niehaus TD et al., BMC Genomics, 2015, 16, 382). Said family includes RiD (reactive intermediate/imine deaminase) proteins. WO 9602567 describes the antitumoral activity of a goat liver extract with perchloric acid (called UK 101) containing protein UK 114. Said protein has a molecular weight of 14.2 kDa and the sequence of 137 amino acids reported in Ceciliani F. et al., FEBS Letters, 1996, 393, 147-150. The native protein UK 114, isolated from the liver of various mammal species, and/or proteins cross-reacting with it, are only present in the cytoplasm of normal cells, whereas they are also present on the cell membrane of malignant tumour cells (Bartorelli A, et al., Int J Oncol. 1996 Mar;8(3):543-8.; Bussolati G, et al., Int J Oncol. 1997 Apr; 10(4):779-85), especially adenocarcinoma, in a percentage exceeding 80%. The expression of UK 114 goat protein in recombinant *E. coli* was described in WO 0063368 and Colombo I et al., Biochim. Biophys. Acta, 1998, 1442, 49-59. More recently, EP 3554639 described a multimeric, in particular trimeric, recombinant form of UK 114 which exhibits increased immunogenic properties useful in the treatment of tumours.

### Description of the invention

It has now been found that Rid/UK114 salmon (*Salmo salar*) proteins, when injected into animals or humans, induce the production of antibodies cytotoxic to tumour cells to an unexpectedly greater extent than found to date with goat (*Capra hircus*) proteins of the same family.

The subject of the invention is therefore UK 114 salmon proteins for use in the treatment and prevention of malignant tumours, and in particular for the immunisation of individuals treated for tumours, adjuvant treatment, and vaccination of individuals at risk of onset or recurrence of malignant tumours.

Rid/UK114 salmon proteins are also useful for passive immunisation with monoclonal antibodies of individuals suffering from or treated for tumours, adjuvant treatment, and treatment of individuals at risk of onset or recurrence of malignant tumours.

The sequence of Rid/UK114 salmon proteins is known.

The amino acid sequences are reported below.
**Amino acid sequence SEQ ID 1 (UK 114 RidA-A):** 
GSHMSSIIRKIINTSKAPAAIGPYSQAVVVDRTMYVSGQLGMDPASGQLVE
GGVQAQTKQALVNMGEILKEAGCGYDSVVKTTVLLADMNDFASVNDVYKTFFS
SSFPARAAYQVAALPRGGLVEIEAVAVLGPLTEVS
**Amino acid sequence SEQ ID 2 (UK 114 RidA-B):**

GSHMAAVQKLFPYTPRAPIRQGIYSQAVVVDRTMYISGQLGLDVASGKLV
EGGVQAQARQALVNMGEILKAAGCGYDNVVKTTVLLADMNDFVNVNDVYKTF
FSKNFPARAAYQVVALPRGGLVEIEAVAVLGPISES.

Form A (UKA) is a homotrimer with a molecular weight of 43.5 kDa. The monomer consists of 139 aa, and has a molecular weight of 14.5 kDa. The hydrodynamic radius of the trimer is 2.9 nm. UKA has an isoelectric point of 5.26. UKA exhibits high conformational stability (Tm about 100°C).

Form B (UKB) is a homotrimer. The monomer consists of 138 aa, and has a molecular weight of 14.7 kDa. The trimer has a molecular weight of 44.1 kDa and a hydrodynamic radius of 2.9 nm.

UKB exhibits greater conformational stability than UKA (Tm about 65°C).

The predicted isoelectric point based on the amino acid sequence is 8.05.

The UK 114 RID A and B salmon (*Salmo salar*) proteins possess a homology of 71% and 61% respectively compared with UK114 Rid human protein, 70% and 62% respectively compared with rabbit Rid protein, and 72% and 64% respectively compared with mouse Rid protein.

Salmon Rid proteins are obtained by recombinant DNA techniques in bacterial, yeast or CHO cells, by known methods. The invention also comprises mutant forms of the proteins which present conservative replacement of amino acids, for example 1 to 10 amino acids or more.

For the recommended therapeutic or preventive uses the salmon protein is administered subcutaneously or intramuscularly in the form of solutions or suspensions in sterile aqueous carriers, optionally conjugated with adjuvants to enhance the immune response.

However, the protein can also be administered by other routes, such as sublingually or topically. The doses for therapeutic, prophylactic and vaccinal applications can range from 1 to 50 mg per administration. A typical vaccination protocol involves 4 administrations, one every fortnight. 20/30 days after the last dose, a booster dose 4 times higher is given. The immunotherapy is monitored with laboratory tests to evaluate the antibody count and cytotoxicity. The treatment can continue until a satisfactory clinical result is achieved (reduction or disappearance of the tumour mass), with boosters given at intervals, depending on the antibody count monitored with laboratory tests, to prevent overimmunisation leading to the risk of immune tolerance. As an alternative to protein administration, the corresponding DNA can be injected by the DNA vaccination technique.

The protein can also be used to prepare monoclonal antibodies which are useful for intramuscular or intravenous passive immunotherapy, and passive seroprophylaxis of immunodepressed patients and/or those who fail to respond to specific immunotherapy.

For example, human monoclonal antibodies are obtained by fusing a humanmouse myeloma K6H6/B5 with lymphocytes transformed with Epstein-Barr virus from patients pre-treated with the protein. IgM-secreting clones which are cytotoxic to tumour cells in the presence of complement are preferred. Alternatively, humanised murine IgM monoclonal antibodies or human IgM-secreting monoclonal antibodies produced in transgenic animals can be used.

The invention is described in detail in the examples set out below.

### Example 1. Production of recombinant salmon proteins (A and B)

For expression of *Salmo salar* recombinant proteins RidA-A and RidA-B in *Escherichia coli,* the expression vector pET-15b is used. The nucleotide sequence encoding RidA-A and RidA-B was obtained by chemical synthesis and inserted in the vector pET-15b. The plasmids obtained express the protein sequences fused to a polyhistidine tag and to a cleavage site recognised by a specific protease at the N terminal. The plasmids created, called pET 15-B-RidA-A and pET15b-RidA-B, were used to transform *Escherichia coli* cells (DH5α).

### Nucleotide sequence SEQ ID 3:

*pet15b-RidA-A (RidA-A Salmo salar* coding sequence in italics):

ATGGGCAGCAGCCATCATCATCATCATCACAGCAGCGGCCTGGTGCCG
CGCGGCAGCCATATGTCTTCGATCATCAGGAAGATAATTAACACCAGTAAAGCGCC
AGCAGCTATCGGGCCGTACAGCCAGGCGGTGGTGGACAGGACCATGTACGT
GTCAGGCCAGCTGGGGATGGACCCTGCCTCTGGTCAGCTGGTGGAAGGAGGAGT
CCAGGCTCAGACCAAACAGGCTCTGGTGAACATGGGGGAGATCCTGAAAGAAGCA
GGGTGTGGATATGACAGTGTCGTGAAAACTACGGTTCTTTTGGCTGACATGAATGA
CTTCGCCAGTGTAAATGACGTCTATAAAACATTTTTCAGCAGTAGCTTCCCAGCGAG
GGCTGCCTACCAGGTCGCTGCTCTGCCCAGGGGTGGGCTTGTAGAGATTGAAGCT
GTGGCTGTTCTAGGCCCTCTGACTGAGGTCTCTTGA

### Nucleotide sequence SEQ ID 4:

*pet15b-RidA-B (RidA-B Salmo salar* coding sequence in italics):

ATGGGCAGCAGCCATCATCATCATCATCACAGCAGCGGCCTGGTGCCG
CGCGGCAGCCATATGGCTGCTGTTCAGAAACTCTTTCCTTATACTCCTAGAGCACC
TATAAGGCAGGGGATTTACAGCCAGGCGGTGGTGGATCGGACGATGTACATC
TCCGGCCAGCTGGGGCTGGACGTGGCCTCAGGGAAGCTGGTGGAGGGAGGGGT
ACAGGCTCAGGCCAGACAGGCTCTGGTCAATATGGGAGAGATCCTGAAAGCAGCT
GGATGTGGTTATGACAATGTCGTCAAGACAACCGTGCTGTTGGCAGACATGAATGA
CTTTGTCAATGTCAACGATGTTTATAAGACATTTTTCAGCAAAAACTTCCCTGCCAGA
GCTGCCTACCAGGTTGTTGCCCTCCCCAGAGGTGGCCTGGTGGAGATCGAGGCTG
TGGCTGTTCTGGGACCCATCTCTGAGTCCTGA

Amino acid sequences SEQ ID 3 and SEQ ID 4 of fusion proteins His-tag-RidA-A and His-Tag-RidA-B are shown below (the segment containing the His-tag up to a thrombin cleavage site is underlined):
**Amino acid sequence SEQ ID 5 (His-tag-RidA-A):** 
MGSSHHHHHHSSGLVPRGSHMSSIIRKIINTSKAPAAIGPYSQAVVVDRTM
YVSGQLGMDPASGQLVEGGVQAQTKQALVNMGEILKEAGCGYDSVVKTTVLL
ADMNDFASVNDVYKTFFSSSFPARAAYQVAALPRGGLVEIEAVAVLGPLTEVS
**Amino acid sequence SEQ ID 6 (His-Tag-RidA-B):**

MGSSHHHHHHSSGLVPRGSHMAAVQKLFPYTPRAPIRQGIYSQAVVVDRT
MYISGQLGLDVASGKLVEGGVQAQARQALVNMGEILKAAGCGYDNVVKTTVL
LADMNDFVNVNDVYKTFFSKNFPARAAYQVVALPRGGLVEIEAVAVLGPISES

To express the RidA-A and RidA-B proteins, the plasmids are transferred from strain DH5α to the expression strain (Rosetta DE3). The transformed cells are cultured in a suitable medium (LB+antibiotics), and protein expression is induced with IPTG for 4 hours at 37°C. This induction time and the temperature of 37°C are ideal for the production of RidA-A and RidA-B proteins, as already demonstrated for UK114 goat protein. Electrophoresis on polyacrylamide gel containing SDS (SDS-PAGE), and staining of the proteins with Coomassie Blue, are conducted to verify the expression of the proteins of interest. The clones tested express the recombinant proteins His-tag-RidA-A or His-tag-RidA-B at a high level, and said proteins are present in the soluble fraction of the cell extract. His-tag-RidA-A or His-tag-RidA-B can be purified by exploiting the affinity of the polyhistidine-tag for nickel resin. The soluble fraction obtained from cells collected 4 hours after induction at 37°C is incubated with the resin to allow the His-tag of the recombinant protein of interest to bind to the nickel conjugated with the resin. The flow-through (everything not bonded to the resin) is then collected, and after various washes the protein is eluted with a high concentration of imidazole which competes with histidine for the nickel bond. The sample, obtained by pooling the eluates with a significant concentration of His-tag-RidA-Ar or His-tag-RidA-B protein, is dialysed in 20 mM Tris-HCl, 300 mM NaCl, pH 7.4. The sample is then incubated with thrombin, a serine protease which specifically recognises the cleavage site at the N-terminal end between the protein sequence and the His-tag sequence, allowing the tag to be removed. The RidA-A or RidA-B proteins are then purified by Size Exclusion Chromatography using a Superdex 75 column, Ge Healthcare, coupled to FPLC (running buffer: saline solution). After checking the fractions eluted by gel filtration on SDS PAGE, the fractions containing the RidA-A or RidA-B proteins are pooled, and the concentration thereof is assayed by UV spectrum and BCA assay.

### Example 2. Activation of innate immunity

A clear demonstration of the different and unexpected activity of salmon Rid proteins, compared with goat Rid protein, derives from analysis of incubation of whole blood or peripheral blood mononuclear cells (PBMC), with different concentrations of the various purified recombinant proteins and subsequent analysis of surface marker expression and cytokine production (CD25, CD69, CD137, CD154, TNF-alpha, IL-1b, IL-6, IL-12, perforin, granzyme A, granzyme B, CD107, interferon-gamma) by various cell subpopulations present and analysed in whole blood and/or PBMC (monocytes, nonclassic "ncMo" monocytes, dendritic cells, low CD14 and CD38 marker expressing cells, CD4+ lymphocytes, CD8+ lymphocytes, gamma-delta lymphocytes, B lymphocytes and NK cells).

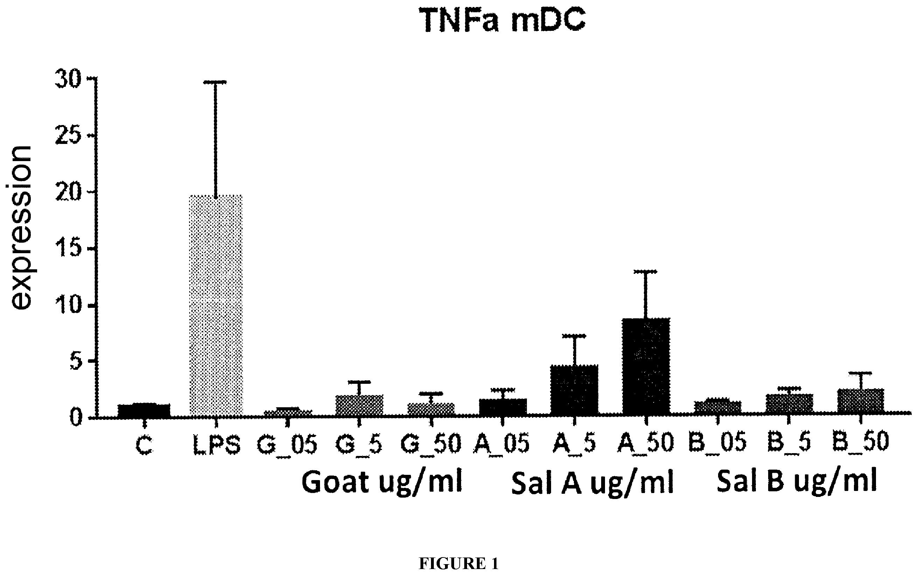

In particular, Figure 1 shows the data obtained by incubating a peripheral blood sample with the indicated concentrations of the various PRP14 protein species (goat [Goat], salmon Form A [Sal A] and salmon Form B [Sal B]), maintaining the untreated sample as control, or the sample treated with Lipopolysaccharide (LPS) as positive control. After 3 hours, the cells underwent flow cytometry analysis, and the monocyte subpopulation was evaluated for intracellular expression of various cytokines as indicated (interleukin 1 beta [IL1b Mono], TNF alpha [TNFa mono], interleukin 6 [IL6 mono], interleukin 12 [IL12 mono]).

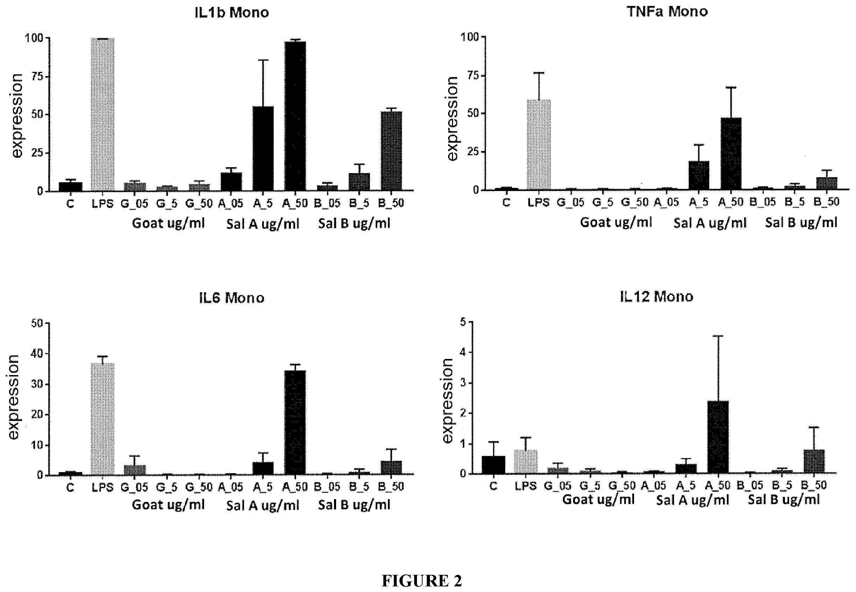

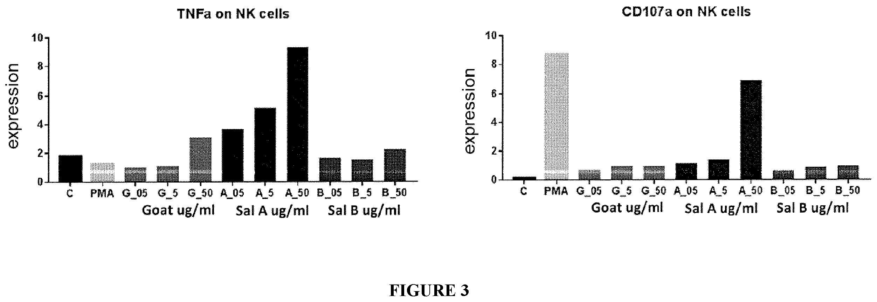

Figure 2 shows the data obtained by incubating a peripheral blood sample with the indicated concentrations of the various PRP14 protein species (goat [Goat], salmon Form A [Sal A] and salmon Form B [Sal B]), maintaining the untreated sample as control, or the sample treated with lipopolysaccharide (LPS) as positive control. After 3 hours, the cells underwent flow cytometry analysis, and the subpopulation of dendritic cells was evaluated for intracellular expression of TNF-alpha as indicated (TNF alpha [TNFa mDC]). Figure 3 shows the data obtained by incubating peripheral blood mononuclear cells with the indicated concentrations of the various PRP14 protein species (goat [Goat], salmon Form A [Sal A] and salmon Form B [Sal B]), maintaining the untreated sample, or a sample treated with phorbol 12-myristate 13-acetate (PMA), as control. After 6 hours, the cells underwent flow cytometry analysis, and the natural killer subpopulation was evaluated for intracellular expression of various cytokines as indicated (TNF alpha [TNFa on NK cells], CD107 [CD107 on NK cells]).

The results shown in Figures 1-3 demonstrate that 3 hours' incubation leads to a dramatic increase in the expression of surface markers and cytokine production, which are characteristic of activation of innate immunity, compared with goat protein, and a greater increase for salmon protein A than salmon protein B. In all cases, while goat protein is inactive or poorly active, salmon proteins A and B induce activation of the innate immune cells (monocytes, "ncMo" monocytes and NK cells) and the other cell types (dendritic cells) also involved in different stages of the adaptive immune response. The data set out in Figures 1-3 therefore demonstrate the greater ability of salmon forms to activate innate immunity on human cells isolated from peripheral blood.

### Example 3. Vaccinal activity

10 micrograms of salmon A protein were injected subcutaneously into mice once a week. 10 days after the fourth injection, 10000 cells of melanoma B16F10 were injected. 14 days after the tumour injection the vaccinal effect was very significant, with a reduction in the mean area of the tumour from 39 +8 mm for the unvaccinated groups to 7 + 8 mm for the vaccinated mice.

### Example 4. Antiserum production

The rabbits and mice received 3 milligrams and 3 micrograms respectively of salmon proteins A and B, with complete Freund's adjuvant, once a fortnight. Each animal was injected subcutaneously at three different points. 10 days after the fourth immunisation, blood samples were taken from the central ear vein of the rabbits and the caudal vein of the mice. The immune response was tested by WB and immunofluorescence flow cytometry on tumour cells, and the hyperimmune sera were tested for their ability to induce cytotoxicity on various tumour cell and normal cell lines. The cell lines listed below were used for said purpose.

| **Positive tumour cells** | **Negative cells** |
|---|---|
| HT-29 (human), LOVO and COLO-684 (human colon carcinoma); KATO III (human gastric carcinoma); | T-47D (human mammary gland); |
| SYSY ND LAN-1 (human neuroblastoma); | 6647 (human sarcoma); |
| MOG-G-UVW (human astrocytoma); | MCF10 (human mammary gland); |
| K562 (human erythroleukaemia); | |
| HL-60 (human promyelocytic leukaemia); | HUVEC (human endothelial cells). |
| TUBE (murine mammary carcinoma); | |
| RPMI 8226 (human multiple myeloma); | |
| HL60: (human leukaemia) | |

The cells were plated in 24-well plates (10,000 cells/well) in high-glucose DMEM (Lonza) 10% FCS (Euroclone) and treated, as soon as they adhered, with 10% sera in low-glucose DMEM (Lonza) plus 1% complement (Sigma). After 48 hours, the Annexin V apoptosis assay was conducted with the Muse^{™} Annexin V & Dead Cell kit (Millipore), according to the manufacturer's instructions.

The cytotoxic activity of the anti-salmon Rid A and B hyperimmune sera was compared with the cytotoxic activity of anti-UK 114 recombinant goat hyperimmune sera (PRP14 Goat).

### Example 5. Comparison of cytotoxic activity: PRP14 goat/salmon A and B

The presence of cytotoxic antibodies in the sera of rabbits immunised with the proteins according to the invention and with goat UK 114 was evaluated. Cell viability was evaluated with the MTT assay (3-(4,5-dimethylthiazol-2-yl)-2,5-diphenyltetrasodium bromide) according to the manufacturer's instructions.

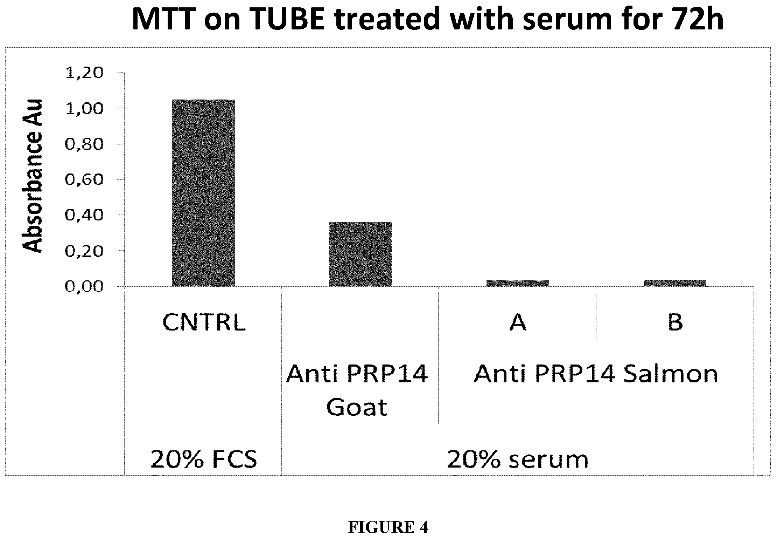

3000 TUBE cells, a cell line cloned from a murine mammary cancer, were plated in 96-well plates in triplicate, treated for 72h with the test sera and incubated for 6h with MTT reagent at 37°C. 100 µl of isopropanol 0.04 N HCl was added to dissolve the crystals, measuring the absorbance at 570 nm. The results are set out in Figure 4.

## Claims

1. UK 114 salmon protein for use in the treatment and prevention of solid and systemic malignancies.

2. Protein for use according to claim 1 wherein the monomeric unit of the protein has the sequence SEQ ID 1 or SEQ ID 2.

3. Protein for use according to claim 1 expressed by *Escherichia coli.*

4. Protein for use according to claim 1 for immunisation of individuals treated for tumours, for adjuvant therapy, and for vaccination of individuals at risk of occurrence or recurrence of malignant tumours.

5. Protein for use according to claim 1 for passive immunisation with monoclonal antibodies of individuals suffering from or treated for tumours, for adjuvant therapy, and for individuals at risk of occurrence or recurrence of malignant tumours.

## Patentansprüche

1. UK 114- Lachsprotein zur Verwendung bei der Behandlung und Vorbeugung von soliden und systemischen bösartigen Tumoren.

2. Protein zur Verwendung gemäß Anspruch 1, wobei die monomere Einheit des Proteins die Sequenz SEQ ID 1 oder SEQ ID 2 aufweist.

3. Protein zur Verwendung gemäß Anspruch 1, das von *Escherichia coli* exprimiert wird.

4. Protein zur Verwendung gemäß Anspruch 1 zur Immunisierung von Personen, die wegen Tumoren behandelt werden, zur adjuvanten Therapie und zur Impfung von Personen, bei denen das Risiko des Auftretens oder Wiederauftretens von bösartigen Tumoren besteht.

5. Protein zur Verwendung gemäß Anspruch 1 zur passiven Immunisierung mit monoklonalen Antikörpern von Personen, die an Tumoren leiden oder wegen Tumoren behandelt werden, zur adjuvanten Therapie und für Personen, bei denen das Risiko des Auftretens oder Wiederauftretens von bösartigen Tumoren besteht.

## Revendications

1. Protéine de saumon UK 114 pour une utilisation dans le traitement et la prévention des tumeurs malignes solides et systémiques.

2. Protéine pour une utilisation selon 1a revendication 1, dans laquelle l'unité monomère de la protéine a la séquence SEQ ID 1 ou SEQ ID 2.

3. Protéine pour une utilisation selon la revendication 1 exprimée par *Escherichia coli.*

4. Protéine pour une utilisation selon 1a revendication 1 pour l'immunisation d'individus traités pour des tumeurs, pour un traitement adjuvant et pour la vaccination d'individus présentant un risque d'apparition ou de récidive de tumeurs malignes.

5. Protéine pour une utilisation selon la revendication 1 pour l'immunisation passive avec des anticorps monoclonaux d'individus souffrant de ou traités pour des tumeurs, pour un traitement adjuvant et pour des individus présentant un risque d'apparition ou de récidive de tumeurs malignes.
